# EUROPEAN PATENT APPLICATION

(11) **EP 4 635 954 A1**
(43) Date of publication of application: **22.10.2025**
(21) Application number: 22968174.7
(22) Date of filing: 15.12.2022
(51) Int. Cl.: C07D 471/04, A61K 31/437, C12N 5/071, A61P 43/00, A61P 35/00

(54) **INDUCER FOR INDUCING MESENCHYMAL-EPITHELIAL TRANSITION AND REPROGRAMMING**

(71) Applicant: Iregene Therapeutics Co., Ltd, Chengdu, Sichuan 610200 (CN)
(72) Inventor: WEI, Jun, Wuhan, Hubei 430000 (CN); CAI, Meng, Wuhan, Hubei 430000 (CN); WEI, Mengya, Wuhan, Hubei 430000 (CN); MAO, Xiaohui, Wuhan, Hubei 430000 (CN); CHEN, Xiping, Wuhan, Hubei 430000 (CN)
(74) Representative: Metida
(86) International application number: PCT/CN2022/139171
(87) International publication number: WO 2024/124464

(57) **Abstract**

The present invention relates to a pyrrolopyridine derivative and its application. The pyrrolopyridine derivative is a compound capable of inducing the conversion of mesenchymal cells into epithelial cells, and has the following structure: 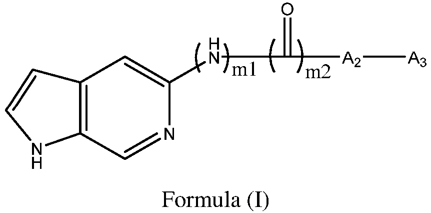 wherein m₁, m₂, A₂, and A₃ are as described herein.

## Description

### Field of the Invention

The present invention relates to the field of medicine, and more specifically to a group of compounds and their application in the process of inducing the conversion of mesenchymal cells into epithelial cells, as well as in cellular reprogramming.

### Background of the Invention

The interconversion between epithelial and mesenchymal cells is a highly conserved and reversible cellular process, wherein polarized and stationary epithelial cells extend filopodia from their basal surface and give rise to migratory mesenchymal cells. Epithelial-to-mesenchymal transition (EMT) and mesenchymal-to-epithelial transition (MET) are well-recognized biological events, that play crucial roles not only in the development of normal tissues and organs, but also in the pathogenesis of various diseases. The phenotypic changes between epithelial and mesenchymal states are classified into epithelial-to-mesenchymal transition (EMT) and mesenchymal-to-epithelial transition (MET), and such morphological transitions are central to the complex remodeling of embryonic and organ structures during gastrulation and organogenesis. They are also recognized as key events in cancer metastasis (Thiery, J.P., Nature Reviews Cancer, 2002; 2:442-454).

During early development in Drosophila, newly formed epithelial germ layers participate in complex morphogenetic movements that drive embryogenesis, for example, ectodermal cells maintain their epithelial phenotype throughout these processes (Tepass, U. Bioessays, 1997; 19:673-682; Tepass, U. & Hartenstein, V. Dev. Biol., 1994; 161:563-596). EMT-MET cycles have also been observed during mesoderm development, where cells invaginate through the ventral furrow to form structures such as the dorsal vessel primordium and Malpighian tubules (Campbell, K. et al., Mech. Dev., 2010; 127:345-357). In most other metazoans, development proceeds through a series of cleavages and progressive assembly of epithelial-like structures from the fertilized egg (Stern, C.D., ed. Gastrulation: From Cells to Embryos, Cold Spring Harbor Laboratory Press, New York, 2004). Accordingly, MET occurs during normal development and is involved in processes such as somatogenesis, kidney development, cardiogenesis, hepatogenesis, and coelom formation (Bin L et al., PLoS One, 2011; 6(2):e17092; Nakajima Y et al., Anat Rec, 2000; 258:119-127). These findings indicate that the underlying mechanisms of MET are similar across different organ morphogenetic events, exhibiting a consistent pattern of upregulation of epithelial-associated genes and downregulation of mesenchymal markers, though each developmental process involves distinct signaling pathways to induce MET and corresponding gene expression changes.

Similarly, embryonic stem cells (ESCs) and induced pluripotent stem cells (iPSCs) undergo EMT and MET during their differentiation into somatic cell types. Conversely, differentiated somatic cells can be reprogrammed to a pluripotent state through a sequential process involving EMT followed by MET, in which MET represents a critical step for acquiring pluripotency (Shu, X. & Pei, D., Curr. Opin. Genet. Dev., 2014; 28:32-37). Studies have shown that MET during reprogramming drives cell fate transitions through coordination with metabolic reprogramming and epigenetic modifications (Wu, J., Ocampo, A., and Belmonte, J.C.I., Cell, 2016; 166:1371-1385). Further studies demonstrated that MET plays an essential role during early somatic cell reprogramming in both mouse embryos and human fibroblasts (Hofding, M.K. and Hyttel, P., Stem Cell Res., 2015; 14:39-53; Subramanyam, D. et al., Nat. Biotechnol., 2011; 29:443-448; Li, R. et al., Cell Stem Cell, 2010; 7:51-63). In this process, the BMP4 signaling pathway plays a pivotal role in initiating MET, by activating intron 2 of CDH1 (encoding E-cadherin) and the promoter region of CLDN4 (encoding claudin-4), thereby promoting MET. Currently, structure-based prediction tools can be used to identify compounds that bind to specific target proteins, and functional screening can identify compounds that upregulate these targets. Meanwhile, various microRNAs (miRNAs) have been found to be highly correlated with the MET process, such as miR-134, miR-145, miR-470, and miR-200c, which exhibit negative regulatory effects on MET (Esther E. Creemers, Anke J. Tijsen, Yigal M. Pinto, Circ Res., 2012; 110(3):483-495; Li, R. et al., Cell Stem Cell, 2010; 7:51-63). Accordingly, inhibitory compounds may be designed based on the structures of these regulatory miRNAs, thereby enabling the positive regulation of the MET process.

### Summary of the Invention

In view of the above, the present invention provides a design based on the structure of the Oct4 protein, as well as the structure of negatively regulating microRNAs (miRNAs) that bind to the Oct4 complex, to develop pyrrolopyridine derivatives capable of simultaneously binding to both the Oct4 protein and the regulatory miRNAs. The pyrrolopyridine derivatives are capable of chemically activating Oct4, hereby regulating the expression of its downstream genes. This avoids the need for viral or other vector-based modulation of Oct4, and further enables a safer and simpler approach to enhancing biological expression through small-molecule compounds.

The present invention provides the use of pyrrolopyridine derivative compounds, which can induce the biological phenomenon of mesenchymal-epithelial transition (MET) in various cell types, causing morphological changes in cells, and inducing the expression of epithelial-associated genes and early reprogramming genes. These compounds serve as potent initiating agents for chemically induced reprogramming.

Specifically, the present invention relates to a compound having a pyrrolopyridine derivative structure represented by Formula (I), or a pharmaceutically acceptable salt thereof, or a solvate, active metabolite, polymorph, ester, optical isomer, or prodrug thereof, a pharmaceutical composition comprising the compound of Formula (I), and its use as a highly selective Oct4 activator in cellular reprogramming.

The present invention provides a compound having the structure of Formula (I): 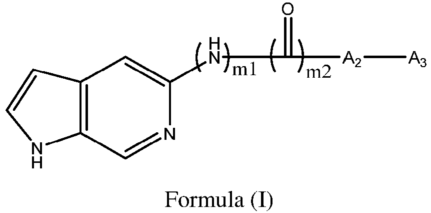 wherein:
m₁ and m₂ are each independently 0 or 1 ;
A₂ is C₁-C₆ alkylene, C₂-C₆ alkenylene, -O(CH₂)q-, -NR₁-, -SO₂-, - (CH₂)ᵥNHSO₂-, or a bond ;
wherein q is 1, 2, 3, or 4; v is 0, 1, or 2; and R₁ is selected from H or C₁-C₄ alkyl ;
A₃ is C₁-C₆ alkyl; C₂-C₆ alkenyl; or C₄-C₆ cycloalkyl, wherein one carbon atom may be substituted with a heteroatom selected from N, O, or S ; 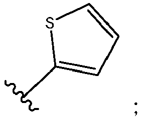 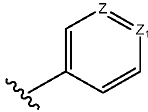 Z and Z₁ are each independently N or CR₂ ; 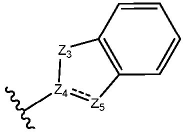 Z₃ is N, O, S, or C=O; when the bond between Z₄ and Z₅ is a single bond, Z₄ is N or CH, and Z₅ is CH₂ or C=O; when the bond between Z₄ and Z₅ is a double bond, Z₄ is C, and Z₅ is CH; or a pharmaceutically acceptable salt, solvate, active metabolite, polymorph, ester, optical isomer, prodrug, or a combination thereof.

In some embodiments: A₂ is -CH₂-, -CH=CH-, -C(CH₃)=CH-, -O(CH₂)-, - O(CH₂)₂-, -NH-, -N(CH₃)-, -SO₂-, -NHSO₂-, -(CH₂)₂NHSO₂-, or a bond.

In some embodiments: A₃ is -CH₃, butenyl,

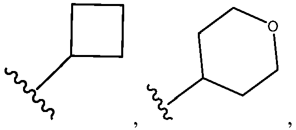

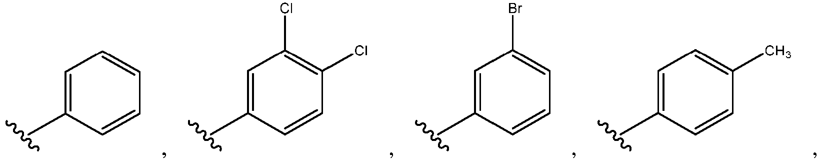

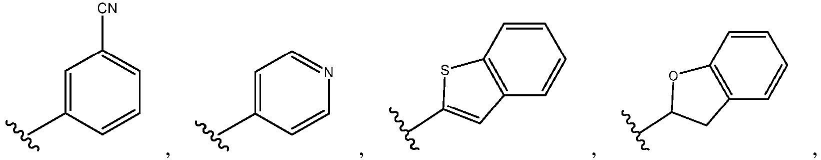

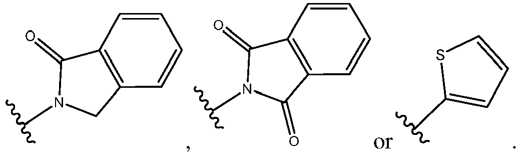

In some embodiments: m₁ is 0, and m₂ is 1 ; A₂ is -N(CH₃)- ; A₃ is

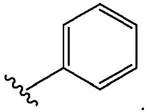

In some embodiments: m₁ is 1, and m₂ is 0 ; A₂ is -CH₂-, -SO₂-, -(CH₂)₂NHSO₂-, or a bond ; A₃ is -CH₃, 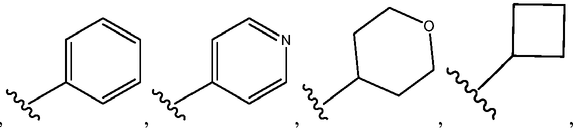 or 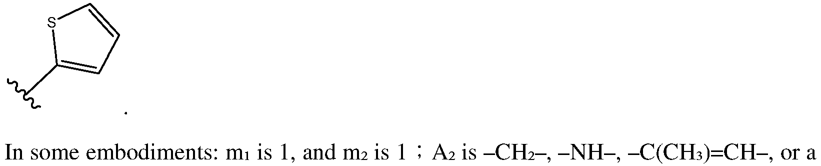 bond ; A₃ is -CH₃, -C(CH₃)=CH-CH₃, 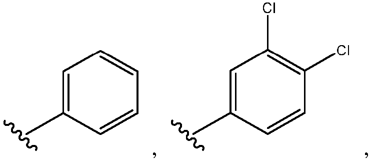 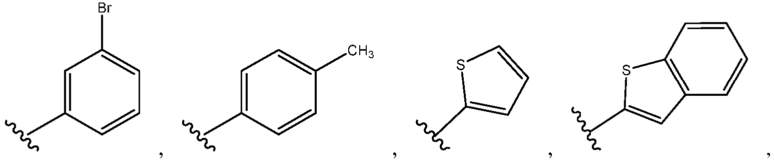 or

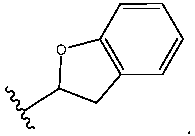

In some embodiments: m₁ is 0, and m₂ is 0 ; A₂ is -CH₂-, -CH=CH-, -O(CH₂)-, -O(CH₂)₂-, or a bond ; A₃ is 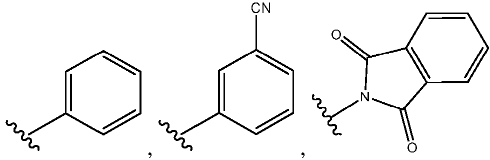 or

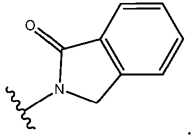

In some embodiments, the compound is:

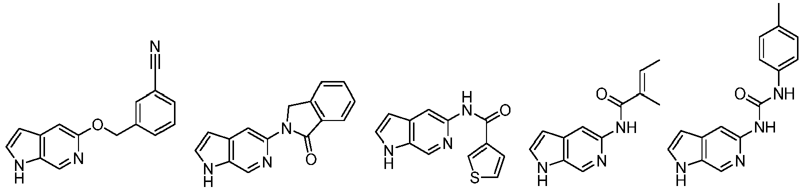

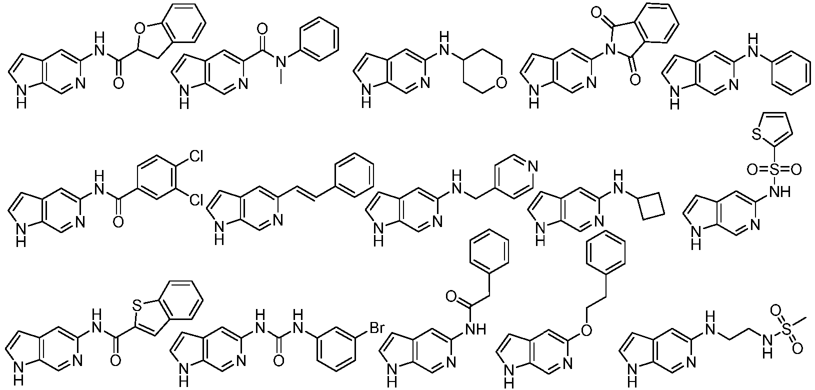

The present invention relates to a pharmaceutical composition, which comprises at least one of the compound according to any of the foregoing embodiments, or a pharmaceutically acceptable salt, solvate, active metabolite, polymorph, ester, optical isomer, prodrug, or a combination of two or more thereof, and at least one pharmaceutically acceptable carrier or excipient.

The present invention further relates to the compound according to any of the foregoing embodiments and/or a pharmaceutically acceptable salt, solvate, active metabolite, polymorph, ester, optical isomer, prodrug, or a combination thereof, for use in the preparation of an agent for inducing the conversion of mesenchymal cells into epithelial cells.

The present invention also relates to a method for activating Oct4, which comprises contacting the compound according to any of the foregoing embodiments and/or a pharmaceutically acceptable salt, solvate, active metabolite, polymorph, ester, optical isomer, prodrug, or a combination thereof with the Oct4 target protein.

### Brief Description of the Figures

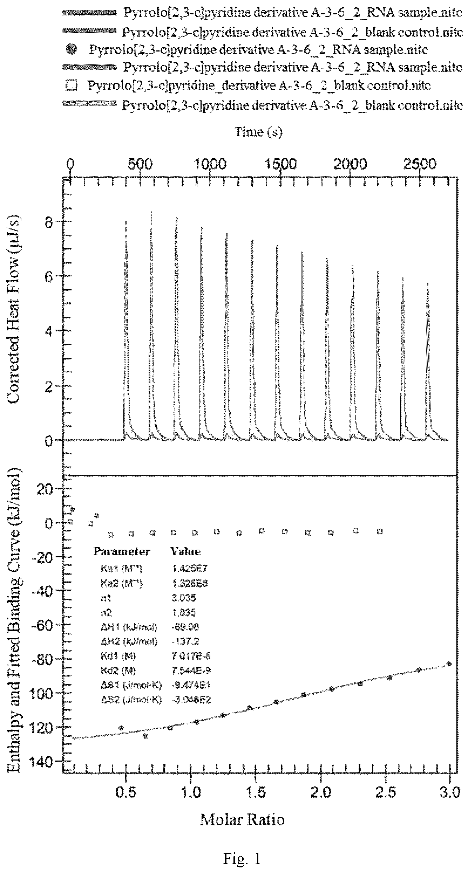

The upper part of Fig. 1 shows the heat changes in the sample cell, where the red peak represents the exothermic binding of miR-145 to A-3-6, and the blue line represents the exothermic binding of buffer without miR-145 to A-3-6. The lower part of Fig. 1 shows the curve fitting of the peak area to a Multiple Sites model, with the calculated nSite values of 3 and 1.8, and the corresponding Kd (M) values of 7.017E-8 and 7.544E-9, respectively; the Kd (M) value for the blank control group is 1.000E-3. The ITC results demonstrate that the small molecule obtained in the present invention is capable of specifically binding to the target.
Fig. 2 shows that compared to the control group treated with vehicle only, cells treated with the pyrrolopyridine derivative alone for 24 hours exhibit the MET phenomenon, as evidenced by a typical epithelial-like morphology (CK represents the control group).
Fig. 3 shows the effect of the pyrrolopyridine derivative on the basal expression of genes such as Oct4 (CK represents the control group).

### Detailed Description of the Invention

In the present invention, the following definitions are applicable:
The term "alkyl" as used herein refers to a saturated hydrocarbon having from 1 to 12 carbon atoms, either in a straight or branched chain. Examples of (C₁-C₆) alkyl include, but are not limited to, methyl, ethyl, propyl, butyl, pentyl, hexyl, isopropyl, isobutyl, sec-butyl, tert-butyl, isopentyl, neopentyl, and isohexyl.

The term "alkenyl" refers to a straight-chain or branched-chain unsaturated hydrocarbon containing 2 to 12 carbon atoms, and having at least one carbon-carbon double bond within the chain. Examples of alkenyl groups include vinyl, allyl, n-but-1-enyl, isobutenyl, pentenyl, or hexenyl.

The term "alkylene" refers to a divalent alkyl group. Any monovalent alkyl group may become an alkylene group by the removal of a second hydrogen atom from the alkyl group. As defined herein, alkylene may be a C₁-C₆ alkylene group. The alkylene group may further be a C₁-C₄ alkylene group. Typical examples of alkylene groups include, but are not limited to: - CH₂-, -CH(CH₃)-, -C(CH₃)₂-, -CH₂CH₂-, - CH₂CH(CH₃)-, - CH₂C(CH₃)₂-, -CH₂CH₂CH₂-, -CH₂CH₂CH₂CH₂-, and the like.

The term "alkenylene" refers to a divalent alkenyl group. Any monovalent alkenyl group may become an alkenylene group by the removal of a second hydrogen atom from the alkenyl group. As defined herein, alkenylene may further be a C₂-C₆ alkenylene group. Typical examples of alkenylene groups include, but are not limited to: - CH=CH-, -CH=C(CH₃)-, -CH=CHCH₂-, -CH=CHCH₂CH₂-, - CH=CHCH₂CH₂CH₂-, -CH=CHCH₂CH₂CH₂CH₂-, and the like.

The term "cycloalkyl" refers to a monocyclic saturated carbon ring containing 3 to 18 carbon atoms. The cycloalkyl group may further be a C₄-C₆ cycloalkyl. Examples of cycloalkyl groups include, but are not limited to, cyclopropyl, cyclobutyl, cyclopentyl, cyclohexyl, cycloheptyl, and cyclooctyl.

The term "halogen" refers to fluorine, chlorine, bromine, or iodine.

The term "cyano" refers to a substituent having a carbon atom triple-bonded to a nitrogen atom (i.e., C≡N).

The term "substituted" as used herein refers to the replacement of one or more hydrogen atoms on a specified atom or group with a group selected from a specified range, provided that the normal valency of the specified atom is not exceeded.

In certain embodiments described in the present invention, a compound of Formula (I) is provided, wherein A₂ is a bond, the structure of the compound of Formula (I) is:

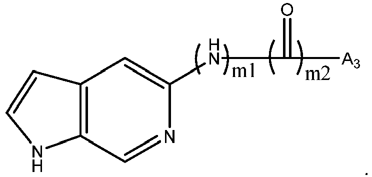

The compounds described herein include, but are not limited to, their optical isomers, racemates, and other mixtures. In such cases, individual enantiomers or diastereomers, i.e., optically active configurations, may be obtained via asymmetric synthesis or by resolution of racemic or diastereomeric mixtures. Resolution of racemic or diastereomeric mixtures can be accomplished by conventional methods, such as crystallization in the presence of a resolving agent, or by chromatographic techniques, such as using a chiral high-performance liquid chromatography (HPLC) column. In addition, the compounds include those having chiral centers in the R- and S-configurations. The compounds also include crystalline forms, including polymorphs and inclusion complexes. Similarly the term "salt" also encompasses all isomers, racemates, other mixtures, R- and S-configurations, tautomers, and crystalline forms of the salts of the compound.

The term "pharmaceutically acceptable salt" refers to salts of the free acid or base forms of the compounds represented by Formula (I), Formula (II), or Formula (III) that are non-toxic, biologically tolerable, and otherwise suitable for administration to a subject. See, e.g., S.M. Berge et al., "Pharmaceutical Salts," J. Pharm. Sci., 1977, 66:1-19; and Handbook of Pharmaceutical Salts: Properties, Selection, and Use, Stahl and Wermuth, Eds., Wiley-VCH and VHCA, Zurich, 2002. Preferably, a pharmaceutically acceptable salt is one that is pharmacologically effective and suitable for contact with patient tissues without causing undue toxicity, irritation, or allergic response. The compounds of Formula (I), (II), or (III) may contain sufficiently acidic or basic functional groups, or both, and accordingly may form salts with various inorganic or organic bases or acids. Examples of pharmaceutically acceptable salts include: sulfates, pyrosulfates, bisulfates, sulfites, bisulfites, phosphates, monohydrogen phosphates, dihydrogen phosphates, metaphosphates, pyrophosphates, hydrochlorides, hydrobromides, hydroiodides, acetates, propionates, decanoates, octanoates, acrylates, formates, isobutyrates, caproates, heptanoates, propargylates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, toluenesulfonates, phenylacetates, phenylpropionates, phenylbutyrates, citrates, lactates, γ-hydroxybutyrates, glycolates, tartrates, methanesulfonates, propanesulfonates, naphthalene-1-sulfonates, naphthalene-2-sulfonates, and mandelates.

A "solvate," such as a "hydrate," is formed through the interaction between a solvent and a compound. The term "compound" includes solvates of the compound, including hydrates. Similarly, the term "salt" includes solvates of the salt, such as hydrates. Suitable solvates are pharmaceutically acceptable solvates, such as hydrates, including monohydrates and hemihydrates.

A "prodrug" may refer to a precursor of the specified compound, which, after administration to a subject, is converted into the compound in vivo by chemical or physiological processes (e.g., solvolysis or enzymatic cleavage), or under physiological conditions (e.g., physiological pH), into a compound of Formula (I). A "pharmaceutically acceptable prodrug" is a prodrug that is non-toxic, biologically tolerable, or otherwise suitable for administration to a subject. Exemplary procedures for selecting and preparing suitable prodrug derivatives are described in, for example, H. Bundgaard (ed.), Design of Prodrugs, Elsevier, 1985.

The term "active metabolite" refers to a pharmaceutically active product resulting from the in vivo metabolism of a compound of Formula (I), (II), or (III), or a salt thereof. The prodrug and active metabolites of a compound can be determined using conventional techniques known or available in the art. See, for example: Bertolini et al., J. Med. Chem. 1997, 40, 2011-2016; Shan et al., J. Pharm. Sci. 1997, 86(7), 765-767; Bagshawe, Drug Dev. Res. 1995, 34, 220-230; Bodor, Adv. Drug Res. 1984, 13, 224-331; Bundgaard, Design of Prodrugs, Elsevier Press, 1985; and Larsen, Design and Application of Prodrugs, in Drug Design and Development (Krogsgaard-Larsen et al., eds., Harwood Academic Publishers, 1991).

A "therapeutically effective amount" refers to an amount of a compound disclosed herein that is sufficient, when administered to a mammal (preferably a human), to treat a disease or condition in the mammal (preferably a human), as defined herein. The amount of the disclosed compound constituting a "therapeutically effective amount" will vary depending on the compound, the disease or condition and its severity, and the age of the mammal being treated, but may be routinely determined by one of ordinary skill in the art in accordance with standard knowledge and the teachings of the present disclosure.

The term "treatment" refers to the administration to an individual of at least one compound disclosed herein and/or at least one pharmaceutically acceptable salt thereof, in order to mitigate (reduce) undesirable physiological changes or a disease, such as the development or spread of inflammation or cancer. The purposes and beneficial or desired clinical outcomes of the present invention include, but are not limited to: alleviation of symptoms, reduction in disease severity, stabilization (i.e., prevention of worsening) of the disease state, delay or slowing of disease progression, improvement or amelioration of the condition, and remission (whether partial or complete) of a detectable or undetectable disease. "Treatment" also means prolonging the survival of the individual compared to the expected survival if left untreated. Individuals in need of treatment include those exhibiting symptoms of or diagnosed with such diseases.

### Pharmaceutical Compositions

The present invention provides pharmaceutical compositions, comprising one or more compounds described herein, or a pharmaceutically acceptable salt or ester thereof, as an active ingredient, and one or more pharmaceutically acceptable excipients or carriers, including inert solid diluents and fillers, and diluents such as sterile aqueous solutions and various organic solvents, penetration enhancers, solubilizers, and adjuvants. The pharmaceutical composition may be administered alone or in combination with other therapeutic agents. Such compositions may be prepared using methods well known in the pharmaceutical arts.

The pharmaceutical composition may be administered in single or multiple doses, via any acceptable route used for agents with similar applications, such as those described in patents and patent applications incorporated herein by reference, including rectal, buccal, intranasal, and transdermal routes, intra-arterial injection, intravenous, intraperitoneal, parenteral, intramuscular, subcutaneous, or oral administration, topical administration, or administration as an inhalant, or via an implantable or coated device such as a stent, including but not limited to an intravascularly inserted polymer-coated column.

The present invention also provides a kit, comprising a pharmaceutical composition that includes a compound of the present invention or a pharmaceutically acceptable salt thereof, and at least one pharmaceutically acceptable carrier.

The term "pharmaceutically acceptable carrier or excipient" refers to a substance that is non-toxic, biologically tolerable, and otherwise biologically suitable for administration to an individual, such as an inert substance, which is added to the pharmaceutical composition or used as a medium, carrier, or diluent to facilitate and be compatible with the administration of the active ingredient. Examples of excipients include calcium carbonate, calcium phosphate, various types of sugars or starches, cellulose derivatives, gelatin, vegetable oils, and polyethylene glycol.

The phrase "a combination of two or more thereof" is to be understood as referring to a combination of two or more of the compound, a pharmaceutically acceptable salt thereof, a pharmaceutically acceptable solvate thereof, a pharmaceutically acceptable active metabolite thereof, a pharmaceutically acceptable polymorph thereof, a pharmaceutically acceptable ester, a pharmaceutically acceptable optical isomer, and a pharmaceutically acceptable prodrug.

### Uses of the Compounds and Compositions

The present invention provides the use of the compounds and compositions thereof, primarily as highly selective activators of Oct4, for activating Oct4 function, by chemically modulating the Oct4 promoter, to regulate the expression of its downstream genes, thereby inducing the conversion of mesenchymal cells into epithelial cells.

List of Abbreviations Used in the Following Examples and Elsewhere in This Disclosure: CH₂Cl₂: dichloromethane; Cs₂CO₃: cesium carbonate; Cu₂SO₄: cuprous sulfate; DCM: dichloromethane; DMAC: N,N-dimethylacetamide; DMF: N,N-dimethylformamide; DIEA: N,N-diisopropylethylamine; DIOX: 1,4-dioxane; EA: acetic acid; Et₃N: triethylamine; EtOH: ethanol; EtOAc: ethyl acetate; FA: formic acid; g: gram; h: hour; HATU: 2-(7-aza-1H-benzotriazol-1-yl)-N,N,N',N'-tetramethyluronium hexafluorophosphate; HBr: hydrobromic acid; (Hbim)BF₄: 1-butylimidazolium tetrafluoroborate; H₂O: water; HAc: acetic acid; H₂O₂: hydrogen peroxide; H₂SO₄: fuming sulfuric acid; KCN: potassium cyanide; K₂CO₃: potassium carbonate; MCA: chloroacetic acid; MeCN: acetonitrile; MeOH: methanol; mg: milligram; mL: milliliter; mmol: millimole; mol: mole; mol/L: mole per liter; m/z: mass-to-charge ratio; N₂: nitrogen; NaBH₃CN: sodium cyanoborohydride; NaNO₂: sodium nitrite; NaOH: sodium hydroxide; Na₂SO₄: sodium sulfate; pH: acidity/basicity; TBN: tert-butyl nitrite; TEA: triethanolamine; THF: tetrahydrofuran; TLC: thin-layer chromatography; *µ*L: microliter; Xylene: xylene.

The following examples, in conjunction with the accompanying figures, are provided to further illustrate the technical means employed in the present invention to achieve the intended objectives. Unless otherwise specified, the experimental methods described in the following examples are conventional methods. Unless otherwise indicated, the raw materials, reagents, and other materials used in the following examples are commercially available products.

### General Synthesis

The general synthetic routes described in the present application may be varied by substituting the starting materials with other reagents of similar structure, to accordingly obtain different products. The following synthetic schemes illustrate multiple examples of how variations in the starting materials may yield corresponding products.

### General Method A-1-n

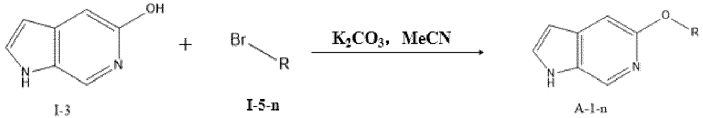

wherein R is 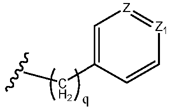 , wherein q is 1, 2, 3, or 4; Z and Z₁ are each independently N or CR₂; R₂ is selected from H, halogen, C₁-C₄ alkyl, or cyano.

### General Method A-3-n

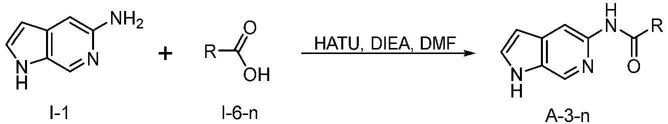

wherein R is a C₂-C₆ alkenyl group; 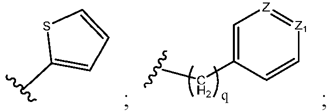 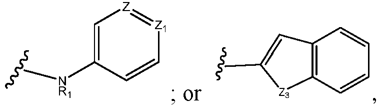 wherein q is 0, 1, 2, 3, or 4; Z and Z₁ are each independently N or CR₂;R₂ is selected from H, halogen, C₁-C₄ alkyl, or cyano; R₁ is selected from H or C₁-C₄ alkyl; Z₃ is N, O, S, or C=O.

### General Method A-4-n

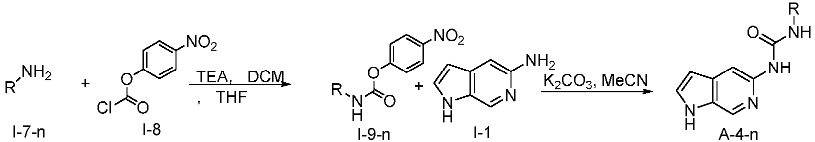

wherein R is 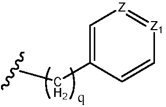 , wherein q is 0, 1, 2, 3, or 4; Z and Z₁ are each independently N or CR₂;R₂ is selected from H, halogen, C₁-C₄ alkyl, or cyano.

### General Method A-6-n

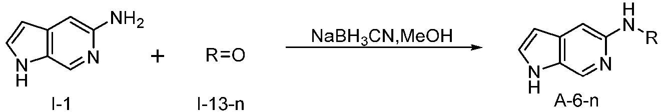

wherein R is a C₄-C₆ cycloalkyl group, wherein one carbon atom may be substituted with a heteroatom selected from N, O, or S; or R=O is 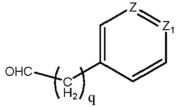 ; wherein q is 0, 1, 2, 3, or 4; Z and Z₁ are each independently N or CR₂; R₂ is selected from H, halogen, C₁-C₄ alkyl, or cyano.

### Intermediate Synthesis

### Intermediate I-3: 1H-pyrrolo[2,3-c]pyridin-5-ol

### Synthetic Route:

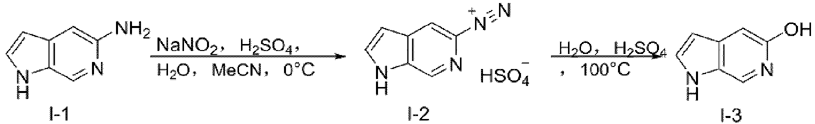

### Step 1: Intermediate 1H-pyrrolo[2,3-c]pyridin-5-diazonium I-2

To a solution of 1H-pyrrolo[2,3-c]pyridin-5-amine I-1 (66.6 mg, 0.5 mmol) were added water (2 mL) and 20% aqueous H₂SO₄ (1 mL). An aqueous solution of sodium nitrite (42 mg, 0.6 mmol in 0.5 mL) and acetonitrile (2 mL) was added dropwise to the mixture under ice cooling, and the reaction was stirred for 30 minutes. To the resulting reaction mixture was added a solution of Cu₂SO₄ (67 mg, 0.3 mmol) in 20% aqueous HBr (0.5 mL) that had been stored at room temperature. The reaction mixture was then stirred at 80 °C for 30 minutes. Ethyl acetate and water were added to the mixture. The organic layer was separated, washed with brine, dried over sodium sulfate, and concentrated under reduced pressure. The residue was purified by silica gel column chromatography (chloroform:methanol = 50:1) to give the hydrogen sulfate salt of 1H-pyrrolo[2,3-c]pyridin-5-diazonium I-2 (94 mg, 78%). LC-MS: m/z = 145.1 [M]⁺.

### Step 2: Intermediate 1H-pyrrolo[2,3-c]pyridin-5-ol I-3

An aqueous solution (1 mL) of the hydrogen sulfate salt of 1H-pyrrolo[2,3-c]pyridin-5-diazonium I-2 (48 mg, 0.2 mmol) was added dropwise into 40% aqueous sulfuric acid (5 mL) at 100 °C, and the resulting mixture was stirred for 10 minutes. Sodium hydroxide was added to the reaction mixture to adjust the pH to approximately 3. Ethyl acetate was then added, and the organic layer was separated, washed with brine, dried over sodium sulfate, and concentrated under reduced pressure. The residue was purified by silica gel column chromatography (chloroform:methanol = 50:1) to afford 1H-pyrrolo[2,3-c]pyridin-5-ol I-3 (20 mg, 77%). LC-MS: m/z = 135.1 [M+H]⁺.

### Example 1: 3-(((1H-pyrrolo[2,3-c]pyridin-5-yl)oxy)methyl)benzonitrile A-1-1

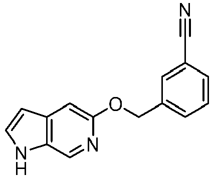

Step: 1H-pyrrolo[2,3-c]pyridin-5-ol I-3 (10 mg, 0.07 mmol) was mixed with 3-(bromomethyl)benzonitrile (20 mg, 0.1 mmol) and K₂CO₃ (138 mg, 1 mmol). Acetonitrile (2 mL) was added, and the mixture was heated to 40 °C and stirred for 10 minutes. Ethyl acetate and water were added to the reaction mixture. The organic layer was separated, dried over sodium sulfate, and concentrated under reduced pressure. The residue was purified by silica gel column chromatography (dichloromethane:methanol = 40:1) to afford 3-(((1H-pyrrolo[2,3-c]pyridin-5-yl)oxy)methyl)benzonitrile A-1-1 (11 mg, 63%). LC-MS: m/z = 250.1 [M+H]⁺

### Example 2: 5-Phenethoxy-1H-pyrrolo[2,3-c]pyridine A-1-2

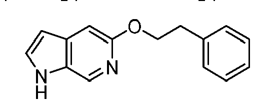

Step: 1H-pyrrolo[2,3-c]pyridin-5-ol I-3 (10 mg, 0.07 mmol) was mixed with (2-bromoethyl)benzene (22 mg, 0.12 mmol) and K₂CO₃ (138 mg, 1 mmol). Acetonitrile (2 mL) was added, and the mixture was heated to 40 °C and stirred for 10 minutes. Ethyl acetate and water were added to the reaction mixture. The organic layer was separated, dried over sodium sulfate, and concentrated under reduced pressure. The residue was purified by silica gel column chromatography (dichloromethane:methanol = 40:1) to afford 5-phenethoxy-1H-pyrrolo[2,3-c]pyridine A-1-2 (13 mg, 78%). LC-MS: m/z = 239.1 [M+H]⁺

### Example 3: (1H-pyrrolo[2,3-c]pyridin-5-yl)isoindolin-1-one A-2

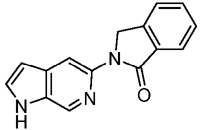

### Synthetic Route:

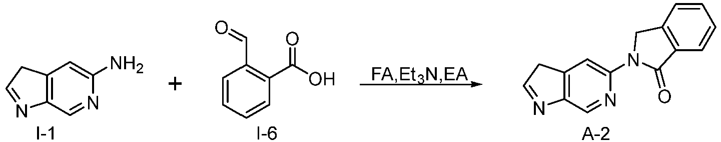

Step:1H-pyrrolo[2,3-c]pyridin-5-amine I-1 (13 mg, 0.1 mmol) was mixed with 2-formylbenzoic acid (18 mg, 0.12 mmol), followed by the addition of formic acid (0.2 mL), triethylamine (1 mL), and ethanol (1 mL). The mixture was heated to 80 °C and stirred for 60 minutes. Ethyl acetate and water were added to the reaction mixture. The organic layer was separated, dried over sodium sulfate, and concentrated under reduced pressure. The residue was purified by silica gel column chromatography (dichloromethane:ethyl acetate = 20:1) to afford (1H-pyrrolo[2,3-c]pyridin-5-yl)isoindolin-1-one A-2 (15 mg, 60%). LC-MS: m/z = 250.3 [M+H]⁺

### Example 4: (1H-pyrrolo[2,3-c]pyridin-5-yl)thiophene-3-carboxamide A-3-1

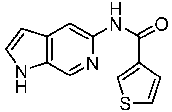

Step:1H-pyrrolo[2,3-c]pyridin-5-amine I-1 (13 mg, 0.1 mmol) was mixed with thiophene-2-carboxylic acid (15 mg, 0.12 mmol) and HATU (2-(7-aza-1H-benzotriazol-1-yl)-N,N,N',N'-tetramethyluronium hexafluorophosphate, 38 mg, 0.1 mmol). N,N-diisopropylethylamine (0.2 mL) and N,N-dimethylformamide (2 mL) were added, and the mixture was stirred at room temperature for 18 hours. Ethyl acetate and water were added to the reaction mixture. The organic layer was separated, dried over sodium sulfate, and concentrated under reduced pressure. The residue was purified by silica gel column chromatography (ethyl acetate:hexane = 3:7) to give N-(1H-pyrrolo[2,3-c]pyridin-5-yl)thiophene-3-carboxamide A-3-1 (10 mg, 41%). LC-MS: m/z = 244.1 [M+H]⁺

### Example 5: (E)-2-Methyl-N-(1H-pyrrolo[2,3-c]pyridin-5-yl)but-2-enamide A-3-2

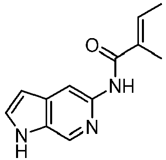

Step:1H-pyrrolo[2,3-c]pyridin-5-amine I-1 (13 mg, 0.1 mmol) was mixed with (E)-2-methyl-2-butenoic acid (12 mg, 0.12 mmol) and HATU (2-(7-aza-1H-benzotriazol-1-yl)-N,N,N',N'-tetramethyluronium hexafluorophosphate, 38 mg, 0.1 mmol).N,N-diisopropylethylamine (0.2 mL) and N,N-dimethylformamide (2 mL) were added, and the reaction mixture was stirred at room temperature for 18 hours. Ethyl acetate and water were then added. The organic layer was separated, dried over sodium sulfate, and concentrated under reduced pressure. The residue was purified by silica gel column chromatography (ethyl acetate:hexane = 3:7) to give (E)-2-methyl-N-(1H-pyrrolo[2,3-c]pyridin-5-yl)but-2-enamide A-3-2 (11 mg, 51%). LC-MS: m/z = 216.1 [M+H]⁺

### Example 6: N-(1H-pyrrolo[2,3-c]pyridin-5-yl)-2,3-dihydrobenzofuran-2-carboxamide A-3-3

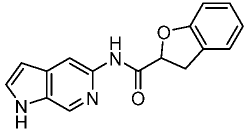

Step:1H-pyrrolo[2,3-c]pyridin-5-amine I-1 (13 mg, 0.1 mmol) was mixed with benzofuran-2-carboxylic acid (19 mg, 0.12 mmol) and HATU (2-(7-aza-1H-benzotriazol-1-yl)-N,N,N',N'-tetramethyluronium hexafluorophosphate, 38 mg, 0.1 mmol). N,N-diisopropylethylamine (0.2 mL) and N,N-dimethylformamide (2 mL) were added, and the mixture was stirred at room temperature for 18 hours. Ethyl acetate and water were added to the reaction mixture. The organic layer was separated, dried over sodium sulfate, and concentrated under reduced pressure. The residue was purified by silica gel column chromatography (ethyl acetate:hexane = 3:7) to afford N-(1H-pyrrolo[2,3-c]pyridin-5-yl)-2,3-dihydrobenzofuran-2-carboxamide A-3-3 (13 mg, 46%). LC-MS: m/z = 280.1 [M+H]⁺

### Example 7: 3,4-Dichloro-N-(1H-pyrrolo[2,3-c]pyridin-5-yl)benzamide A-3-4

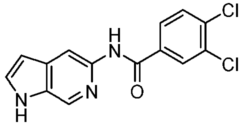

Step: 1H-pyrrolo[2,3-c]pyridin-5-amine I-1 (13 mg, 0.1 mmol) was mixed with 3,4-dichlorobenzoic acid (23 mg, 0.12 mmol) and HATU (2-(7-aza-1H-benzotriazol-1-yl)-N,N,N',N'-tetramethyluronium hexafluorophosphate, 38 mg, 0.1 mmol). N,N-diisopropylethylamine (0.2 mL) and N,N-dimethylformamide (2 mL) were added, and the mixture was stirred at room temperature for 18 hours. Ethyl acetate and water were then added. The organic layer was separated, dried over sodium sulfate, and concentrated under reduced pressure. The residue was purified by silica gel column chromatography (ethyl acetate:hexane = 3:7) to afford 3,4-dichloro-N-(1H-pyrrolo[2,3-c]pyridin-5-yl)benzamide A-3-4 (13 mg, 43%). LC-MS: m/z = 306.0 [M+H]⁺

### Example 8: N-(1H-pyrrolo[2,3-c]pyridin-5-yl)benzo[b]thiophene-2-carboxamide A-3-5

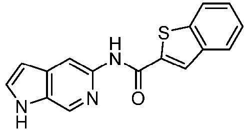

Step:1H-pyrrolo[2,3-c]pyridin-5-amine I-1 (13 mg, 0.1 mmol) was mixed with benzo[b]thiophene-2-carboxylic acid (21 mg, 0.12 mmol) and HATU (2-(7-aza-1H-benzotriazol-1-yl)-N,N,N',N'-tetramethyluronium hexafluorophosphate, 38 mg, 0.1 mmol). N,N-diisopropylethylamine (0.2 mL) and N,N-dimethylformamide (2 mL) were added, and the mixture was stirred at room temperature for 18 hours. Ethyl acetate and water were added to the reaction mixture. The organic layer was separated, dried over sodium sulfate, and concentrated under reduced pressure. The residue was purified by silica gel column chromatography (ethyl acetate:hexane = 3:7) to afford N-(1H-pyrrolo[2,3-c]pyridin-5-yl)benzo[b]thiophene-2-carboxamide A-3-5 (14 mg, 48%). LC-MS: m/z = 294.1 [M+H]⁺

### Example 9: 2-Phenyl-N-(1H-pyrrolo[2,3-c]pyridin-5-yl)acetamide A-3-6

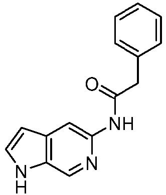

Step:1H-pyrrolo[2,3-c]pyridin-5-amine I-1 (13 mg, 0.1 mmol) was mixed with 2-phenylacetic acid (16 mg, 0.12 mmol) and HATU (2-(7-aza-1H-benzotriazol-1-yl)-N,N,N',N'-tetramethyluronium hexafluorophosphate, 38 mg, 0.1 mmol). N,N-diisopropylethylamine (0.2 mL) and N,N-dimethylformamide (2 mL) were added, and the mixture was stirred at room temperature for 18 hours. Ethyl acetate and water were then added. The organic layer was separated, dried over sodium sulfate, and concentrated under reduced pressure. The residue was purified by silica gel column chromatography (ethyl acetate:hexane = 3:7) to afford 2-phenyl-N-(1H-pyrrolo[2,3-c]pyridin-5-yl)acetamide A-3-6 (12 mg, 48%). LC-MS: m/z = 252.1 [M+H]⁺

### Example 10: 1-(1H-pyrrolo[2,3-c]pyridin-5-yl)-3-(p-tolyl)urea A-4-1

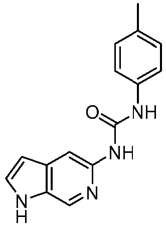

### Step 1: p-Nitrophenyl p-tolyl carbamate I-9-1

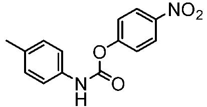

p-Toluidine (21 mg, 0.2 mmol) was mixed with 4-nitrophenyl chloroformate (48 mg, 0.24 mmol), followed by the addition of triethanolamine (0.2 mL), dichloromethane (1 mL), and tetrahydrofuran (2 mL). The mixture was stirred at room temperature for 18 hours. After completion, ethyl acetate and water were added. The organic layer was separated, dried over sodium sulfate, and concentrated under reduced pressure. The residue was purified by silica gel column chromatography (dichloromethane:hexane = 5:1) to afford p-nitrophenyl p-tolyl carbamate I-9-1 (42 mg, 77%). LC-MS: m/z = 273.1 [M+H]⁺

### Step 2: 1-(1H-pyrrolo[2,3-c]pyridin-5-yl)-3-(p-tolyl)urea A-4-1

To a mixture of the above-obtained p-nitrophenyl p-tolyl carbamate I-9-1 (27 mg, 0.1 mmol), 1H-pyrrolo[2,3-c]pyridin-5-amine I-1 (16 mg, 0.12 mmol), and K₂CO₃ (0.2 g, 1.45 mmol) was added acetonitrile (3 mL). The reaction mixture was heated to 40 °C and stirred for 4 hours. Upon completion of the reaction (monitored by TLC), the mixture was concentrated under vacuum.The crude residue was sequentially washed with dichloromethane, EtOAc, and MeOH (each 1 mL). Finally, the product was recrystallized from warm EtOAc to afford the pure compound 1-(1H-pyrrolo[2,3-c]pyridin-5-yl)-3-(p-tolyl)ureaA-4-1 (22 mg, 83%). LC-MS: m/z = 267.1 [M+H]⁺

### Example 11: 1-(3-Bromophenyl)-3-(1H-pyrrolo[2,3-c]pyridin-5-yl)urea A-4-2

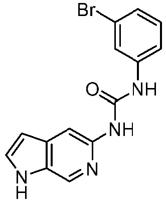

### Step 1: 4-Nitrophenyl (3-bromophenyl)carbamate I-9-2

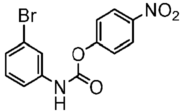

3-Bromoaniline (34 mg, 0.2 mmol) was mixed with 4-nitrophenyl chloroformate (48 mg, 0.24 mmol), followed by the addition of triethanolamine (0.2 mL), dichloromethane (1 mL), and tetrahydrofuran (2 mL). The mixture was stirred at room temperature for 18 hours. Ethyl acetate and water were added to the reaction mixture. The organic layer was separated, dried over sodium sulfate, and concentrated under reduced pressure. The residue was purified by silica gel column chromatography (dichloromethane:hexane = 5:1) to afford 4-nitrophenyl (3-bromophenyl)carbamate I-9-2 (46 mg, 68%). LC-MS: m/z = 338.1 [M+H]⁺

### Step 2: 1-(3-Bromophenyl)-3-(1H-pyrrolo[2,3-c]pyridin-5-yl)urea A-4-2

To a mixture of the above-obtained 4-nitrophenyl (3-bromophenyl)carbamate I-9-2 (34 mg, 0.1 mmol),v1H-pyrrolo[2,3-c]pyridin-5-amine I-1 (16 mg, 0.12 mmol), and K₂CO₃ (0.2 g, 1.45 mmol) was added acetonitrile (3 mL), and the mixture was stirred at 40 °C for 4 hours. Upon completion (monitored by TLC), the reaction mixture was concentrated under vacuum. The resulting crude residue was sequentially washed with dichloromethane, EtOAc, and MeOH (each 1 mL). The final product was recrystallized from warm EtOAc to afford the pure compound 1-(3-bromophenyl)-3-(1H-pyrrolo[2,3-c]pyridin-5-yl)urea A-4-2 (25 mg, 75%). LC-MS: m/z = 331.2 [M+H]⁺

### Example 12: N-Methyl-N-phenyl-1H-pyrrolo[2,3-c]pyridine-5-carboxamide A-5

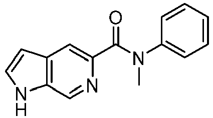

### Synthetic Route:

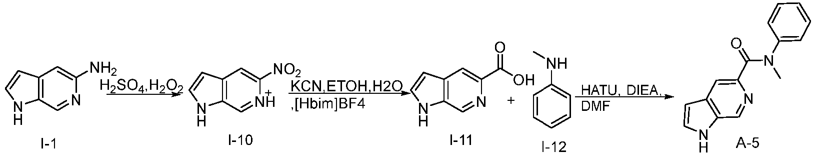

### Step 1: 5-Nitro-1H-pyrrolo[2,3-c]pyridine I-10

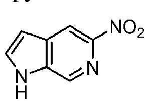

To a solution of H₂O₂ (240 mg, 2.2 mmol) in fuming sulfuric acid (500 µL) was added dropwise a solution of 1H-pyrrolo[2,3-c]pyridin-5-amine I-1 (40 mg, 0.3 mmol) in concentrated sulfuric acid (100 µL), while maintaining the reaction temperature at 0 °C. The reaction mixture was then stirred at 10-25 °C for 3 hours. After stirring, 40% aqueous NaOH was added at 0-5 °C to adjust the pH to 11-12. The resulting mixture was extracted with ethyl acetate. The combined organic layers were washed with saturated aqueous sodium chloride, dried over Na₂SO₄, and filtered. The solvent was removed under reduced pressure to yield the desired product 5-nitro-1H-pyrrolo[2,3-c]pyridine I-10 (39 mg, 80%). LC-MS: m/z = 164.0 [M+H]⁺

### Step 2: 1H-pyrrolo[2,3-c]pyridine-5-carboxylic acid I-11

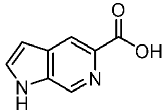

5-Nitro-1H-pyrrolo[2,3-c]pyridine I-10 (39 mg, 0.24 mmol) was mixed with KCN (195 mg, 3 mmol), 1-butylimidazolium tetrafluoroborate (3 mg, 0.014 mmol), EtOH (2 mL), and H₂O (2 mL). The mixture was heated to 80 °C and stirred for 19 hours. After cooling, 10 mL of water was added, and the mixture was extracted with CH₂Cl₂ (3 × 5 mL) and ether (3 × 10 mL). The aqueous layer was acidified with HCl to pH 1-2 and then extracted again with ether (3 × 10 mL). The combined extracts were treated with magnesium sulfate (3 g) and activated charcoal (1 g) and stirred for 5 hours. The solids were filtered off, and the filtrate was evaporated. The residue was crystallized from a suitable solvent to afford 1H-pyrrolo[2,3-c]pyridine-5-carboxylic acid I-11 (16 mg, 41%). LC-MS: m/z = 163.0 [M+H]⁺

### Step 3: N-Methyl-N-phenyl-1H-pyrrolo[2,3-c]pyridine-5-carboxamide A-5

1H-pyrrolo[2,3-c]pyridine-5-carboxylic acid I-11 (16 mg, 0.1 mmol) was mixed with N-methylaniline (13 mg, 0.12 mmol) and HATU (38 mg, 0.1 mmol). N,N-diisopropylethylamine (0.2 mL) and N,N-dimethylformamide (2 mL) were added, and the reaction mixture was stirred at room temperature for 18 hours. Ethyl acetate and water were added to the reaction mixture. The organic layer was separated, dried over sodium sulfate, and concentrated under reduced pressure. The residue was purified by silica gel column chromatography (ethyl acetate:hexane = 3:7), and the solvent was removed under reduced pressure to yield N-methyl-N-phenyl-1H-pyrrolo[2,3-clpyridine-5-carboxamide A-5 (18 mg, 72%). LC-MS: m/z = 252.1 [M+H]⁺

### Example 13: N-(Tetrahydro-2H-pyran-4-yl)-1H-pyrrolo[2,3-c]pyridin-5-amine A-6-1

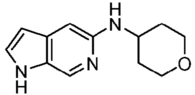

1H-pyrrolo[2,3-c]pyridin-5-amine I-1 (13 mg, 0.1 mmol) was mixed with tetrahydro-4H-pyran-4-one (12 mg, 0.12 mmol) in methanol (2 mL), and the mixture was stirred at room temperature for 2 hours. Then, sodium cyanoborohydride (20 mg, 0.3 mmol) was added, and the reaction was stirred for another 2 hours at room temperature. Aqueous NaOH solution (10 mL, 0.3 mol/L) was then added to the mixture. The resulting mixture was extracted with dichloromethane (DCM, 3 × 20 mL). The combined organic phases were dried over anhydrous Na₂SO₄, filtered, and concentrated under vacuum. The crude product was purified by silica gel column chromatography (petroleum ether:ethyl acetate = 5:1). The solvent was removed under reduced pressure to afford N-(tetrahydro-2H-pyran-4-yl)-1H-pyrrolo[2,3-c]pyridin-5-amine A-6-1 (18 mg, 83%). LC-MS: m/z = 218.1 [M+H]⁺

### Example 14: N-(Pyridin-4-ylmethyl)-1H-pyrrolo[2,3-c]pyridin-5-amine A-6-2

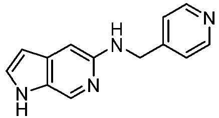

1H-pyrrolo[2,3-c]pyridin-5-amine I-1 (13 mg, 0.1 mmol) was mixed with isonicotinaldehyde (13 mg, 0.12 mmol) in methanol (2 mL), and the mixture was stirred at room temperature for 2 hours. Sodium cyanoborohydride (20 mg, 0.3 mmol) was then added, and the reaction was stirred for an additional 2 hours at room temperature. Aqueous NaOH solution (10 mL, 0.3 mol/L) was added to the reaction mixture. The resulting mixture was extracted with dichloromethane (DCM, 3 × 20 mL). The combined organic layers were dried over anhydrous Na₂SO₄, filtered, and concentrated under reduced pressure. The crude product was purified by silica gel column chromatography (petroleum ether:ethyl acetate = 5:1). After removing the solvent under reduced pressure, N-(pyridin-4-ylmethyl)-1H-pyrrolo[2,3-c]pyridin-5-amine A-6-2 was obtained (16 mg, 71%). LC-MS: m/z = 225.1 [M+H]⁺

### Example 15: N-Cyclobutyl-1H-pyrrolo[2,3-c]pyridin-5-amine A-6-3

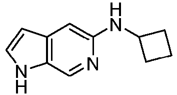

1H-pyrrolo[2,3-c]pyridin-5-amine I-1 (13 mg, 0.1 mmol) was mixed with cyclobutanone (8 mg, 0.12 mmol) in methanol (2 mL), and the mixture was stirred at room temperature for 2 hours. Sodium cyanoborohydride (20 mg, 0.3 mmol) was then added, and the reaction was stirred for another 2 hours at room temperature. Aqueous NaOH solution (10 mL, 0.3 mol/L) was added to the reaction mixture. The resulting mixture was extracted with dichloromethane (DCM, 3 × 20 mL). The combined organic phases were dried over anhydrous Na₂SO₄, filtered, and concentrated under reduced pressure. The crude product was purified by silica gel column chromatography (petroleum ether:ethyl acetate = 5:1). The solvent was removed under reduced pressure to yield N-cyclobutyl-1H-pyrrolo[2,3-c]pyridin-5-amine A-6-3 (15 mg, 80%). LC-MS: m/z = 188.1 [M+H]⁺

### Example 16: 2-(1H-pyrrolo[2,3-c]pyridin-5-yl)isoindoline-1,3-dione A-7

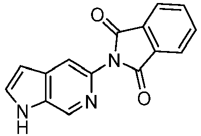

### Synthetic Route:

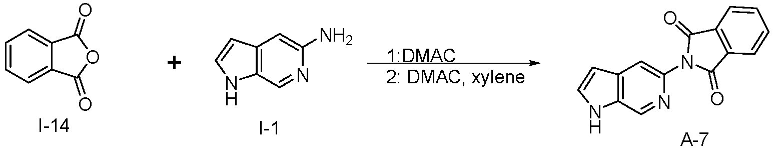

Step:1H-pyrrolo[2,3-c]pyridin-5-amine I-1 (13 mg, 0.1 mmol) was mixed with isobenzofuran-1,3-dione (18 mg, 0.12 mmol) in N,N-dimethylacetamide (2 mL), and the mixture was stirred at room temperature for 24 hours. Then, xylene (1 mL) was added and the mixture was stirred in an oil bath at 140 °C for 48 hours. Upon completion (monitored by TLC), the mixture was filtered to remove the insoluble catalyst, washed with acetone, and dried. The organic phase was concentrated under reduced pressure to obtain the crude product, which was washed with water and recrystallized from ethanol. The crude product was further purified by silica gel column chromatography (CH₂Cl₂:n-hexane = 1:1), and the solvent was removed under reduced pressure to afford 2-(1H-pyrrolo[2,3-c]pyridin-5-yl)isoindoline-1,3-dione A-7 (20 mg, 76%). LC-MS: m/z = 264.1 [M+H]⁺

### Example 17: N-Phenyl-1H-pyrrolo[2,3-c]pyridin-5-amine A-8

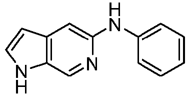

### Synthetic Route:

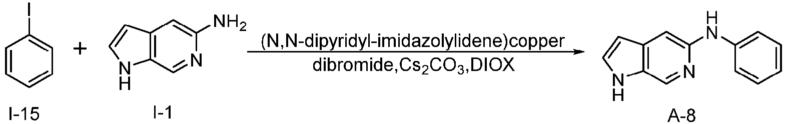

Step:1H-pyrrolo[2,3-c]pyridin-5-amine I-1 (13 mg, 0.1 mmol), iodobenzene (24 mg, 0.12 mmol), bis(µ-iodo)(N,N'-di-pyridylimidazolidinyl)copper(II) bromide (10 mg, 0.023 mmol), and cesium carbonate (100 mg, 0.3 mmol) were mixed in 1,4-dioxane (5 mL). The reaction mixture was stirred in an oil bath at 170 °C for 12 hours. After cooling, aqueous NaOH solution (10 mL, 0.3 mol/L) was added. The resulting mixture was extracted with dichloromethane (DCM, 3 × 20 mL). The combined organic layers were dried over anhydrous Na₂SO₄, filtered, and concentrated under reduced pressure. The crude product was purified by silica gel column chromatography (petroleum ether:ethyl acetate = 5: 1).The solvent was removed under reduced pressure to give N-phenyl-1H-pyrrolo[2,3-c]pyridin-5-amine A-8 (19 mg, 91%). LC-MS: m/z = 210.1 [M+H]⁺

### Example 18: (E)-5-Styryl-1H-pyrrolo[2,3-c]pyridine A-9

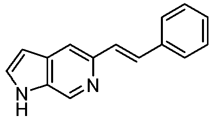

### Synthetic Route:

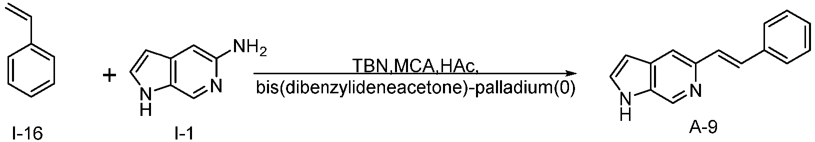

Step: 1H-pyrrolo[2,3-c]pyridin-5-amine I-1 (13 mg, 0.1 mmol) was mixed with styrene (13 mg, 0.12 mmol) and bis(dibenzylideneacetone)palladium(0) (5 mg, 0.0087 mmol). tert-Butylnitrite (0.5 mL), chloroacetic acid (0.5 mL), and acetic acid (3 mL) were added, and the reaction mixture was stirred in an oil bath at 50 °C for 2 hours. After completion, aqueous NaOH solution (10 mL, 0.3 mol/L) was added. The mixture was extracted with dichloromethane (DCM, 3 × 20 mL). The combined organic layers were dried over anhydrous Na₂SO₄, filtered, and concentrated under reduced pressure. The crude product was purified by silica gel column chromatography (petroleum ether:ethyl acetate = 5:1). The solvent was removed under reduced pressure to afford (E)-5-styryl-1H-pyrrolo[2,3-c]pyridine A-9 (15 mg, 68%). LC-MS: m/z = 221.1 [M+H]⁺

### Example 19: N-(1H-pyrrolo[2,3-c]pyridin-5-yl)thiophene-2-sulfonamide A-10

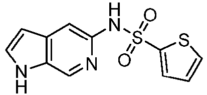

### Synthetic Route:

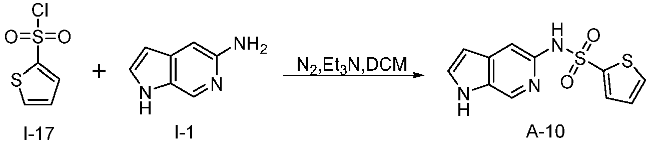

Step: 1H-pyrrolo[2,3-c]pyridin-5-amine I-1 (13 mg, 0.1 mmol) was mixed with thiophene-2-sulfonyl chloride (22 mg, 0.12 mmol), followed by the addition of triethylamine (0.5 mL) and dichloromethane (3 mL). The reaction was stirred at room temperature under a nitrogen atmosphere for 24 hours. Ethyl acetate and water were then added to the reaction mixture. The organic layer was separated, dried over sodium sulfate, and concentrated under reduced pressure. The residue was purified by silica gel column chromatography (ethyl acetate:hexane = 3:7).After removal of solvent under reduced pressure, N-(1H-pyrrolo[2,3-c]pyridin-5-yl)thiophene-2-sulfonamide A-10 was obtained (14 mg, 50%). LC-MS: m/z = 280.0 [M+H]⁺

### Example 20: N-(2-((1H-pyrrolo[2,3-c]pyridin-5-yl)amino)ethyl)methanesulfonamide A-11

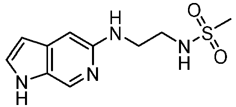

### Synthetic Route:

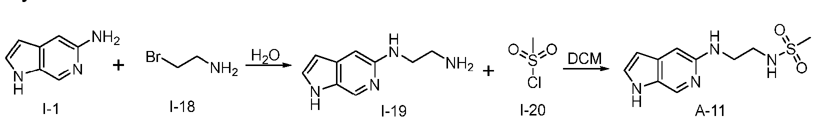

### Step 1: N¹-(1H-pyrrolo[2,3-c]pyridin-5-yl)ethane-1,2-diamine I-19

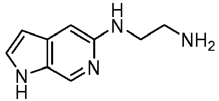

1H-pyrrolo[2,3-c]pyridin-5-amine I-1 (26 mg, 0.2 mmol) was mixed with 2-bromoethylamine I-18 (29 mg, 0.24 mmol) in water (5 mL), and the mixture was stirred in an oil bath at 95 °C for 18 hours. After completion of the reaction, ethyl acetate and water were added. The organic layer was separated, dried over sodium sulfate, and concentrated under reduced pressure. The residue was purified by flash chromatography on silica gel (CH₂Cl₂:methanol-ammonia),and the solvent was removed under reduced pressure to afford N¹-(1H-pyrrolo[2,3-c]pyridin-5-yl)ethane-1,2-diamine I-19 (24 mg, 68%). LC-MS: m/z = 177.1 [M+H]⁺

### Step 2: N-(2-((1H-pyrrolo[2,3-c]pyridin-5-yl)amino)ethyl)methanesulfonamide A-11

The above-obtained N¹-(1H-pyrrolo[2,3-c]pyridin-5-yl)ethane-1,2-diamine I-19 (24 mg, 0.13 mmol) was mixed with methanesulfonyl chloride I-20 (28 mg, 0.16 mmol) in dichloromethane (5 mL), and the mixture was stirred in an ice-water bath for 24 hours. After completion, ethyl acetate and water were added. The organic layer was separated, dried over sodium sulfate, and concentrated under reduced pressure. The residue was purified by silica gel column chromatography (ethyl acetate:hexane = 3:7). After solvent removal under vacuum, N-(2-((1H-pyrrolo[2,3-c]pyridin-5-yl)amino)ethyl)methanesulfonamide A-11 was obtained (12 mg, 36%). LC-MS: m/z = 255.1 [M+H]⁺

### Example 21: The present invention utilized two compound prediction methods to evaluate pyrrolo[2,3-c]pyridine derivatives, as described below:

### Method 1: Protein Structure-Based Docking Prediction

Molecular docking was performed using AutoDock Vina and LeDock software to simulate the interaction between the pyrrolo[2,3-c]pyridine derivatives from Examples 1 to 20 and the Oct4 target protein, with each docking simulation generating 10 docking conformations. The binding energy and ligand efficiency of the optimal docking pose for each pyrrolo[2,3-c]pyridine compound with the Oct4 target protein were calculated, and the compounds were ranked based on the combined docking results. The specific docking results are presented in Table 1. The second and third columns in the table represent the binding free energies calculated by AutoDock Vina and LeDock, respectively. More negative values indicate stronger binding affinities between the small molecule ligands and the target protein. The fourth column shows the ligand efficiency values calculated using LeDock, with larger absolute values suggesting greater potential biological activity of the small molecule. In the present invention, the binding energy levels of the disclosed compounds were predicted using the independent algorithms of both software tools. The predicted values indicate that all the compounds exhibit binding energies significantly lower (i.e., more negative) than the threshold value of 3, which was established based on the characteristics of the target protein.

**Table 1. Predicted Binding Energies of Pyrrolo[2,3-c]pyridine Derivatives with the Target Sequence in the Present Invention**

| Compound | AD_vina_binding_energy (kcal/mol) | Ledock_Energy (kcal/mol) | Ledock_Ligand_efficiency |
|---|---|---|---|
| A-1-1 | -6.2 | -5.05 | -0.27 |
| A-1-2 | -5.9 | -4.92 | -0.27 |
| A-2 | -6.4 | -4.50 | -0.24 |
| A-3-1 | -5.3 | -4.78 | -0.28 |
| A-3-2 | -5.6 | -5.03 | -0.31 |
| A-3-3 | -6.4 | -4.68 | -0.22 |
| A-3-4 | -6.5 | -4.85 | -0.24 |
| A-3-5 | -6.6 | -4.85 | -0.23 |
| A-3-6 | -6 | -4.81 | -0.25 |
| A-4-1 | -6 | -4.80 | -0.24 |
| A-4-2 | -5.9 | -5.24 | -0.26 |
| A-5 | -6 | -4.26 | -0.22 |
| A-6-1 | -5.6 | -4.54 | -0.28 |
| A-6-2 | -5.8 | -4.95 | -0.29 |
| A-6-3 | -5.3 | -4.39 | -0.31 |
| A-7 | -6.7 | -4.67 | -0.23 |
| A-8 | -5.85 | -4.57 | -0.29 |
| A-9 | -6.2 | -4.70 | -0.28 |
| A-10 | -5.3 | -5.36 | -0.30 |
| A-11 | -4.85 | -5.62 | -0.33 |

### Method 2: mir-RNA Structure-Based Docking Prediction

The binding affinity between miR-145 and small molecules was predicted using a novel computational tool, RLDOCK. RLDOCK is a ligand-RNA binding prediction model based on physical deformation theory. It employs a newly developed global multi-step binding site search algorithm that improves both computational efficiency and stability, enabling the accurate prediction of unknown RNA binding sites. During multi-step calculations, RLDOCK performs an exhaustive scan of potential binding sites on the miR-145 structure and explores possible binding poses of the small molecules at each site. It applies a minimal Lennard-Jones potential scoring function to evaluate and rank all possible binding configurations, with the results summarized in Table 2. The docking scores are presented as negative values, where a larger absolute value indicates stronger ligand-RNA affinity. The final RLDOCK score represents a composite value integrating multiple energy functions employed by the algorithm. As the number of validated RNA targets remains limited, there is currently no established computational threshold to definitively distinguish actual interactions. Accordingly, candidate compounds were prioritized for further validation based on predicted binding affinity rankings.

**Table 2: Predicted Binding Energies Between Pyrrolo[2,3-c]pyridine Derivatives and the Target miR-RNA**

| Compound | RLDOCK_SF-h_SCORE(Based_on_Energy) |
|---|---|
| A-1-1 | -16.35 |
| A-1-2 | -22.13 |
| A-2 | -14.17 |
| A-3-1 | -17.16 |
| A-3-2 | -17.55 |
| A-3-3 | -11.27 |
| A-3-4 | -15.99 |
| A-3-5 | -6.05 |
| A-3-6 | -18.14 |
| A-4-1 | -20.06 |
| A-4-2 | -17.58 |
| A-5 | -17.28 |
| A-6-1 | -16.28 |
| A-6-2 | -20.83 |
| A-6-3 | -1.24 |
| A-7 | -5.41 |
| A-8 | -19.77 |
| A-9 | -12.25 |
| A-10 | -18.08 |
| A-11 | -17.71 |

Based on docking predictions involving the Oct4 protein structure and the miR-145 RNA structure, the pyrrolo[2,3-c]pyridine derivatives demonstrated strong binding affinities in both models, suggesting that these compounds may possess the ability to enhance Oct4 expression. To validate this hypothesis, isothermal titration calorimetry (ITC) and cellular experiments were conducted to assess the effect of the pyrrolo[2,3-c]pyridine derivatives on Oct4 expression levels. These studies served to confirm the role of pyrrolo[2,3-c]pyridine derivatives as highly selective activators of Oct4.

### Example 22: Validation of Small Molecule-Nucleic Acid Interaction Using Isothermal Titration Calorimetry (ITC)

Isothermal titration calorimetry (ITC) operates based on the principle of power compensation and allows accurate determination of the enthalpy change associated with molecular interactions across a broad concentration range using a single titration experiment. It measures the heat change upon binding and provides a complete thermodynamic profile of nucleic acid-ligand interactions. Quantitative thermodynamic characterization of the complex is essential for understanding the molecular basis of the interaction between the microRNA miR-145 and the small molecule compound. In this invention, the Nano-ITC calorimeter from Waters Corporation was used to validate in vitro the interaction between nucleic acid and small molecule compounds. The experimental procedure involved placing 300 µL of singlestranded miR-145 solution at a concentration of 50 µM into a temperature-controlled sample cell, which was thermally coupled via a thermocouple circuit to a reference cell containing an equal volume of deionized water. As an example, the small molecule A-3-6 was prepared at a concentration of 500 µM and a volume of 50 µL, and loaded into the injection syringe as the ligand. The heat change in the sample cell was measured and balanced against the reference cell, appearing as either endothermic or exothermic peaks. The built-in software was used to fit the interaction model between the two reactants, enabling direct calculation of the binding enthalpy (ΔH), heat capacity at constant pressure (ΔCp), number of binding sites (n), and association constant (Ka). From these parameters, relevant thermodynamic and kinetic data were derived. The upper panel of Figure 1 shows the heat change in the sample cell: the red trace represents the exothermic binding of A-3-6 to miR-145, while the blue trace represents the binding of A-3-6 to the buffer without miR-145. The lower panel of Figure 1 shows the fitted curve using a Multiple Sites model. The calculated number of binding sites (nSite) was 3 and 1.8, corresponding to dissociation constants (Kd) of 7.017×10⁻⁸ M and 7.544×10⁻⁹ M, respectively, while the control group (buffer only) showed a Kd of 1.000×10⁻³ M. The ITC results demonstrated that the small molecules disclosed in the present invention are capable of specifically binding to the intended target.

### Example 23: Validation of MET-Induced Morphological Changes in Cells by Small Molecule Compounds

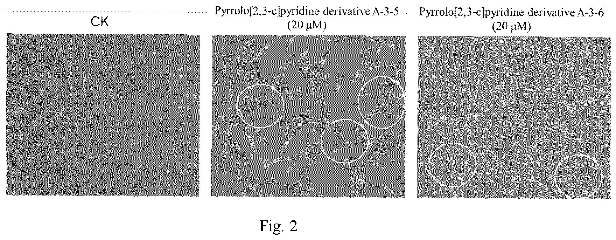

Human mesenchymal cells were cultured in T25 flasks by seeding 4 × 10⁵ cells using serum-free Dulbecco's Modified Eagle Medium/Nutrient Mixture F-12 (DMEM-F12). Each culture was treated with 20 µM of the pyrrolo[2,3-c]pyridine derivative, and cells were incubated at 37 °C in a 5% CO₂ atmosphere. As shown in Figure 2, compared with the control group without the addition of pyrrolo[2,3-c]pyridine derivatives, treatment with the pyrrolo[2,3-c]pyridine derivative alone for 24 hours induced a mesenchymal-to-epithelial transition (MET) phenotype, with cells exhibiting characteristic epithelial morphology. These morphological observations demonstrate that the pyrrolo[2,3-c]pyridine derivatives are capable of rapidly inducing mesenchymal-to-epithelial transition in human mesenchymal cells.

### Example 24: Verification of Transcriptional Expression Changes Induced by Small Molecule Treatment

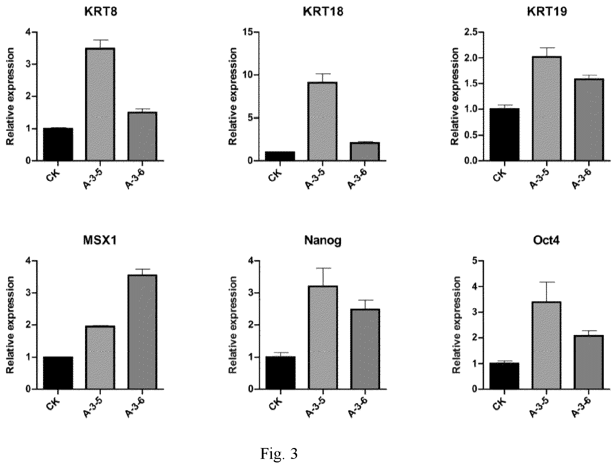

The purpose of the present invention is to induce mesenchymal-to-epithelial transition (MET) using specific compounds. A critical function of such activators is the enhancement of gene expression associated with MET-induced morphological changes. According to previous literature reports, in evaluating the function of the small molecules described in this invention, the structural protein family KRT (keratin) and regulatory genes such as the MSX family were used as molecular markers for MET. Results showed that pyrrolo[2,3-c]pyridine and its derivatives are capable of inducing the MET process. Upregulation of genes downstream of Oct4 also serves as an indicator for validating the biological activity of the compounds in the present invention. In particular, expression of the early reprogramming gene Nanog was used to further confirm the Oct4 activation capability of pyrrolo[2,3-c]pyridine derivatives. Human mesenchymal cells were cultured in T25 flasks by seeding 4 × 10⁵ cells in serum-free DMEM/F12 medium. Cells were treated with 50 nM of each pyrrolo[2,3-c]pyridine derivative and incubated at 37 °C with 5% CO₂. On day 3, total RNA was extracted using the RNeasy Mini or Micro Kit (QIAGEN). 1 µg of RNA was reverse-transcribed into cDNA using the SuperScript III First-Strand Synthesis System (Invitrogen). Quantitative PCR was carried out using SYBR Premix Ex Taq (TaKaRa) on a Thermal Cycler Dice Real-Time System (TaKaRa). Beta-actin was used as an internal control. All data were analyzed using the delta-Ct method. Each group was tested in triplicate and analyzed by statistical analysis of variance (ANOVA). The primer sequences used for detecting marker gene expression are listed in Table 3. As shown in Figure 3, the expression levels of Oct4 and related genes were significantly increased in cells treated with the above pyrrolo[2,3-c]pyridine derivatives compared to the untreated control group.

**Table 3. QPCR Primer Sequences for Compound-Induced Gene Expression Analysis**

| | |
|---|---|
| Oct4-F | CCATGCATTCAAACTGAGGT |
| Oct4-R | CCTTTGTGTTCCCAATTCCTT |
| Nanog-F | ACCTCAGCTACAAACAGGTGAA |
| Nanog-R | AAAGGCTGGGGTAGGTAGGT |
| KRT8-F | CAGAAGTCCTACAAGGTGTCCA |
| KRT8-R | CTCTGGTTGACCGTAACTGCG |
| KRT18-F | TCGCAAATACTGTGGACAATGC |
| KRT18-R | GCAGTCGTGTGATATTGGTGT |
| KRT19-F | ACCAAGTTTGAGACGGAACAG |
| KRT19-R | CCCTCAGCGTACTGATTTCCT |
| MSX1-F | AGAAGATGCGCTCGTCAAA |
| MSX1-R | GGCTTACGGTTCGTCTTGT |
| βActin-F | GGCCGAGGACTTTGATTGCACA |
| βActin-R | GGGCACGAAGGCTCATCATTCAA |

## Claims

1. An inducer for converting mesenchymal cells into epithelial cells, **characterized in that** the inducer comprises a compound having the structure of Formula (I) : 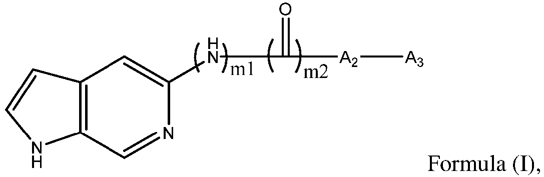 wherein:
m₁ and m₂ are each independently 0 or 1;
A₂ is C₁-C₆ alkylene, C₂-C₆ alkenylene, -O(CH₂)q-, -NR₁-, -SO₂-, - (CH₂)_{y}NHSO₂-, or a bond, wherein q is 1, 2, 3, or 4, v is 0, 1, or 2, and R₁ is selected from H or C₁-C₄ alkyl;
A₃ is C₁-C₆ alkyl, C₂-C₆ alkenyl, or C₄-C₆ cycloalkyl, wherein one carbon atom may be substituted by a heteroatom selected from N, O, or S; 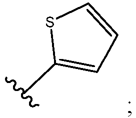 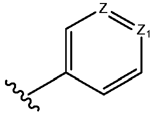 Z and Z₁ are each independently N or CR₂, wherein R₂ is selected from H, halogen, C₁-C₄ alkyl, or cyano; 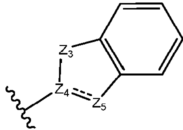 Z₃ is N, O, S, or C=O; when the bond between Z₄ and Z₅ is a single bond, Z₄ is N or CH, and Z₅ is CH₂ or C=O; when the bond between Z₄ and Z₅ is a double bond, Z₄ is C, and Z₅ is CH;
or a pharmaceutically acceptable salt, solvate, active metabolite, polymorph, ester, optical isomer, or prodrug thereof.

2. The inducer according to claim 1, **characterized in that** in the compound: A₂ is -CH₂-, -CH=CH-, -C(CH₃)=CH-, -O(CH₂)-, -O(CH₂)₂-, -NH-, -N(CH₃)-, -SO₂-, -NHSO₂-, -(CH₂)₂NHSO₂-, or a bond.

3. The inducer according to claim 2, **characterized in that** in the compound:
A₃ is -CH₃, butenyl,

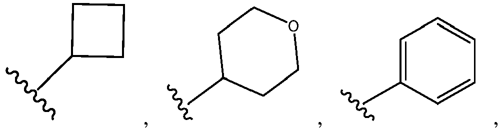

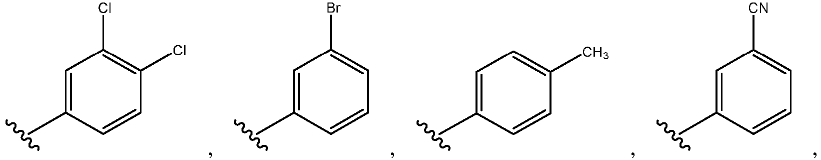

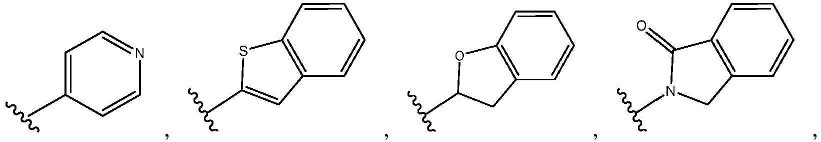

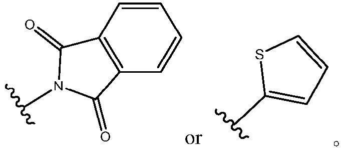

4. The inducer according to claim 3, **characterized in that** in the compound:
m₁ is 0, m₂ is 1;
A₂ is -N(CH₃)-;
A₃ is

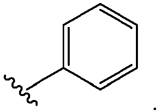

5. The inducer according to claim 3, **characterized in that** in the compound:
m₁ is 1, m₂ is 0 ;
A₂ is -CH₂-, -SO₂-, -(CH₂)₂NHSO₂-, or a bond ;
A₃ is -CH₃,

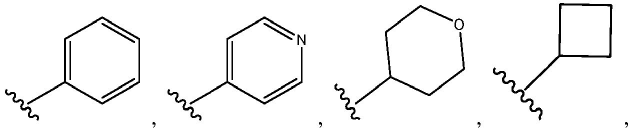

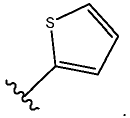

6. The inducer according to claim 3, **characterized in that** in the compound:
m₁ is 1, m₂ is 1 ;
A₂ is -CH₂-, -NH-, -C(CH₃)=CH-, or a bond ;
A₂ is -CH₄, -C(CH₃)=CH-CH_{3,} 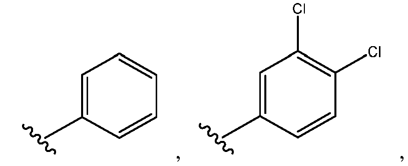 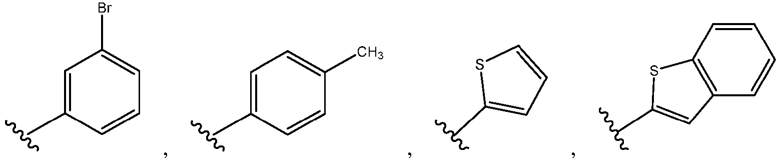 or

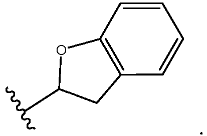

7. The inducer according to claim 3, **characterized in that** in the compound:
m₁ is 0, m₂ is 0 ;
A₂ is -CH₂-, -CH=CH-, -O(CH₂)-, -O(CH₂)₂-, or a bond ;
A₃ is

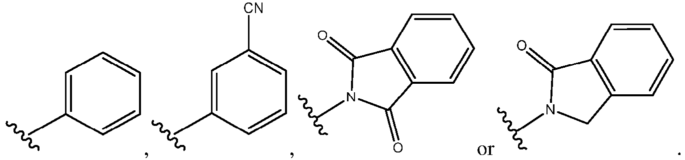

8. The inducer according to claim 1, **characterized in that** the compound is selected from:

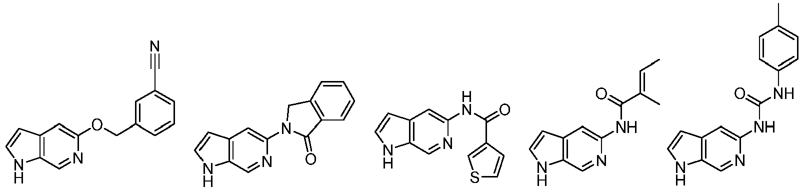

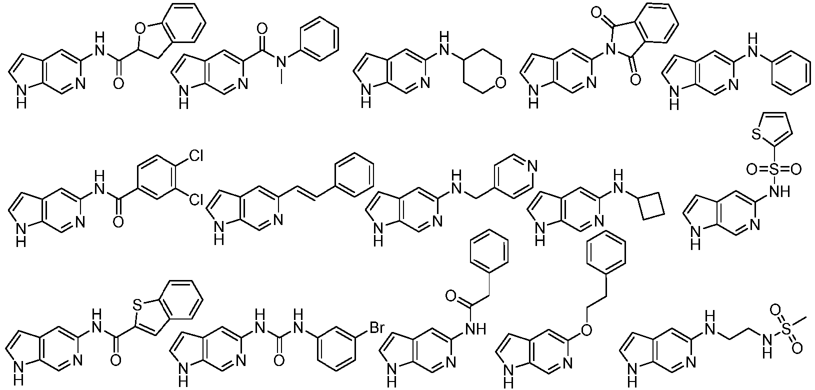

9. A pharmaceutical composition comprising: the inducer according to any one of claims 1 to 8, or a pharmaceutically acceptable salt, solvate, active metabolite, polymorph, ester, optical isomer, prodrug, or a combination of two or more thereof; and at least one pharmaceutically acceptable carrier or excipient.

10. Use of the inducer according to any one of claims 1 to 8 and/or a pharmaceutically acceptable salt, solvate, active metabolite, polymorph, ester, optical isomer, prodrug, or a combination thereof in the preparation of an agent for inducing the conversion of mesenchymal cells into epithelial cells.
